# EUROPÄISCHE PATENTSCHRIFT

(11) **EP 3 463 182 B1**
(45) Veröffentlichungstag und Bekanntmachung des Hinweises auf die Patenterteilung: **14.09.2022**
(21) Anmeldenummer: 17729750.4
(22) Anmeldetag: 29.05.2017
(51) Int. Cl.: A61F 2/00, A61F 5/00

(54) **MEDIZINISCHE EINRICHTUNG ZUM VERENGEN ODER ABSPERREN EINES KÖRPERKANALS**
MEDICAL DEVICE FOR NARROWING OR CLOSING AN ANATOMICAL CHANNEL
DISPOSITIF MÉDICAL POUR RÉTRÉCIR OU FERMER UN CANAL CORPOREL

(30) Priorität: 03.06.2016 AT 2742016
(43) Veröffentlichungstag der Anmeldung: 10.04.2019
(73) Patentinhaber: A.M.I. Agency for Medical Innovations GmbH, 6800 Feldkirch (AT)
(72) Erfinder: HOHLRIEDER, Martin, 6840 Götzis (AT); GILLEN, Thomas, 79713 Bad Säckingen (DE)
(74) Vertreter: Torggler & Hofmann Patentanwälte - Rankweil
(86) Internationale Anmeldenummer: PCT/AT2017/000042
(87) Internationale Veröffentlichungsnummer: WO 2017/205883

(56) Entgegenhaltungen:
- EP-A1- 1 832 253
- EP-A1- 2 123 238
- US-A1- 2015 359 617

## Beschreibung

Die Erfindung bezieht sich auf eine medizinische Einrichtung zum Verengen oder Absperren eines Körperkanals, umfassend ein Bandteil, das um das den Körperkanal umgebende Körpergewebe legbar ist und zu einem eine Durchtrittsöffnung für das Körpergewebe umschließenden Ring schließbar ist, wobei das Bandteil eine Hohlkammer aufweist und wobei durch Einbringen eines Fluids in die Hohlkammer die Durchtrittsöffnung verkleinerbar ist, und eine Pumpeinheit zur Förderung des Fluids, welche einen von einer elektronischen Steuerung der Einrichtung ansteuerbaren elektrischen Antrieb aufweist, wobei von der elektronischen Steuerung ein dem Druck des Fluids in der Hohlkammer entsprechender oder von diesem abhängender Druckwert erfassbar ist.

Medizinische Einrichtungen zum Verengen oder Absperren eines Körperkanals werden u. a. als künstliche (Schließ)-Muskeln zur Unterstützung oder zum Ersatz von geschwächten natürlichen Muskeln im menschlichen oder tierischen Körper eingesetzt. Beispiele für Einsatzbereiche derartiger Einrichtungen sind Analbänder zum Verschließen eines, gegebenenfalls künstlichen, Anus und künstliche Schließmuskel zur Behandlung von Inkontinenz zum Verschließen der Urethra (=Harnröhre). Weitere Einsatzgebiete sind z.B. Magenbänder zum Verengen des Gastro-Intestinal-Traktes oder Bänder zum Verschließen eines Gangs für Gallenflüssigkeit. Derartige medizinische Einrichtungen werden auch als Cuff, Manschette oder artifizieller Sphinkter bezeichnet.

Die Hohlkammer kann vom Benutzer bei Bedarf entleert werden, um die Querschnittsfläche der Durchtrittsöffnung zu vergrößern und im Körperkanal enthaltene Stoffe und/oder Flüssigkeiten passieren zu lassen. Z.B. bei der Anwendung als künstlicher Schließmuskel für die Urethra erfolgt häufig ein anschließendes automatisches Verschließen des Körperkanals durch Rückpumpen von Fluid (evtl. über ein Drosselventil) in die Hohlkammer der Einrichtung. Eine Pumpe zum Pumpen von Fluid wird bei einem solchen künstlichen Harnsphinkter für männliche Patienten üblicherweise in das Skrotum implantiert. Das Pumpen von Fluid aus der Hohlkammer kann dann durch Druck auf einen flexiblen Teil der Pumpe erfolgen. Das Rückpumpen von Fluid in die Hohlkammer kann durch ein federelastisches Element der Pumpe erfolgen. Die Durchtrittsöffnung der medizinischen Einrichtung kann häufig auch durch eine bewusste Handlung des Benutzers wieder verkleinert werden, also durch eine manuelle Betätigung der Pumpe.

Z.B. aus der US 5,478,305 A geht eine medizinische Einrichtung hervor, welche in dieser Schrift als Cuff bezeichnet wird und zur Behandlung von Harn- oder Stuhlinkontinenz eingesetzt werden kann. Der Cuff ist aus Silikon hergestellt. Durch die Befüllung des Cuffs mit Fluid steigt der Druck im Hohlraum des Cuffs an und verschließt den Körperkanal. Beispiele für Magenbänder gehen aus der EP 1 389 453 B1 hervor.

Bekannt sind weiters implantierbare medizinische Einrichtungen, durch welche ein flüssiges Medikament an einen gewünschten Ort innerhalb des Körpers dosiert werden kann. Aus der US 5,976,109 A ist eine derartige Einrichtung bekannt, bei welcher ein Behälter mit einer flexiblen Wand, beispielsweise in Form eines Balges, vorgesehen ist, wobei ein Deckelteil des Behälters zur Dosierung des Medikaments von einem elektrischen Antrieb verstellt wird. Der Behälter bildet somit gleichzeitig ein Reservoir für das Medikament und einen Teil einer Pumpe, durch welche das Medikament an die gewünschte Stelle im Körper gefördert wird.

Eine Einrichtung der eingangs genannten Art geht aus der DE 100 13 519 A1 hervor. Es handelt sich um eine implantierbare Sphinkterprothese zur Anwendung bei Harninkontinenz. Zur Förderung des Fluids dient ein elektrisch angetriebenes Pumpteil. Zur Energieversorgung ist hier ein extrakorporales Versorgungs- und Steuergerät vorgesehen, von welchem der Antrieb des Pumpteils drahtlos mit elektrischer Energie versorgt werden kann. Im Bereich des Bandteils ist ein Drucksensor vorgesehen, von dem der vom Bandteil auf die Harnröhre wirkende Druck ermittelt wird. Dieser Drucksensor kann zum Kalibrieren des Systems, also zum Einstellen des Schließdrucks genützt werden. Der Drucksensor kann auch, bei Verwendung einer Pumpe mit umkehrbarer Förderrichtung oder eines Umschaltventils, zur Einstellung der Förderrichtung, d.h. von der Sphinkterprothese in ein Reservoir oder umgekehrt, herangezogen werden.

Aus der EP 1 832 253 A1 geht ein Magenband mit einem Drucksensor zur Ermittlung des Fluiddrucks im Magenband hervor. Im Weiteren ist in dieser Schrift ein Luftdrucksensor offenbart, dessen Messwert zur Korrektur des vom Drucksensor ermittelten Werts des Fluiddrucks herangezogen wird. Dadurch kann die Genauigkeit der Druckmessung erhöht und ein tatsächlicher Druck im Magenband ermittelt und an einer Anzeigeeinheit ausgegeben werden. Eine Fachperson kann dann eine genaue Einstellung des Drucks im Magenband vornehmen, wobei Fluid händisch, z.B. mittels einer Spritze, über einen im Körper implantierten Port zugeführt oder entnommen werden kann.

Medizinische Einrichtungen zur Beeinflussung des Durchflusses des Darmtrakts und zur Stimulation einer Kontraktion des Darmes sind in der US 2015/0359617 A1 gezeigt.

Aufgabe der Erfindung ist es, eine Einrichtung der eingangs genannten Art bereitzustellen, die einen verbesserten Betrieb ermöglicht. Erfindungsgemäß gelingt dies durch eine Einrichtung mit den Merkmalen des Anspruchs 1.

Die Einrichtung gemäß der Erfindung weist einen Luftdrucksensor auf. Von der elektronischen Steuerung ist somit ein von diesem Luftdrucksensor ausgegebener Luftdruckwert erfassbar, der dem Atmosphärendruck entspricht. Insbesondere kann von der Steuerung unter Heranziehung von zu verschiedenen Zeitpunkten erfassten Druckwerten, die dem jeweiligen Druck des Fluids in der Hohlkammer entsprechen oder von diesem abhängen, und von erfassten Luftdruckwerten, die dem jeweiligen Atmosphärendruck entsprechen, eine Änderung des Fluidvolumens im System festgestellt und gegebenenfalls der eingestellte Druck für das Fluid daran angepasst werden und/oder im Falle einer Überschreitung eines oberen oder unteren Grenzwerts für das festgestellte Flüssigkeitsvolumen eine Warnung ausgegeben werden.

Eine solche Änderung des Flüssigkeitsvolumens kann beispielsweise durch Osmose oder durch ein Leck hervorgerufen werden.

Durch die erfindungsgemäße Einrichtung können vorteilhafterweise optimale Druckverhältnisse an der Harnröhre erzielt werden.

Ein dem Druck des Fluids in der Hohlkammer entsprechender Druckwert kann in einer möglichen Ausbildungsform mittels eines im Fluid angeordneten Drucksensors erfasst werden.

Auch eine Anordnung eines Drucksensors an einer die Durchtrittsöffnung begrenzenden Oberfläche des Bandteils ist denkbar und möglich. Ein von einem solchen Drucksensor erfasster Druckwert hängt jedenfalls vom Druck des Fluids in der Hohlkammer ab und kann vorzugsweise zum Druck des Fluids in der Hohlkammer zumindest im Wesentlichen proportional sein.

Ein Druckwert könnte auch mittels eines Dehnmessstreifens gemessen werden, der auf eine Membran geklebt ist, die sich aufgrund von Druckschwankungen verformt. Die Membran begrenzt einen Raum in welchem sich das Fluid befindet. Beispielsweise könnte die Membran auch von einem Abschnitt des Bandteils selbst gebildet werden. Es wird durch eine solche Membran mit einem aufgeklebten Dehnmessstreifen ein Drucksensor ausgebildet, der an das Fluid angrenzt. Auch ein durch einen auf diese Weise ausgebildeten Drucksensor erfasster Druckwert hängt jedenfalls vom Druck des Fluids in der Hohlkammer ab und kann vorzugsweise zum Druck des Fluids in der Hohlkammer zumindest im Wesentlichen proportional sein.

Je nach eingesetztem elektrischem Antrieb könnte ein dem Druck des Fluids in der Hohlkammer entsprechender oder von diesem abhängender Druckwert auch aus der Stromaufnahme des elektrischen Antriebs der Pumpeinrichtung abgeleitet werden.

Eine vorteilhafte Ausführungsform sieht vor, dass die Pumpeinheit ein Pumpteil aufweist, das einen mit dem Fluid befüllten Aufnahmeraum besitzt, dessen Volumen mittels des Antriebs veränderbar ist. Beispielsweise kann das Pumpteil ein von einem durch ein Bodenteil und ein Deckelteil verschlossener Balg sein. Auch eine Ausbildung des Pumpteils in Form einer Kolben-Zylinder-Einheit ist denkbar und möglich. Der Drucksensor zur Erfassung des Drucks des Fluids in der Hohlkammer des Bandteils kann in einem solchen Aufnahmeraum eines Pumpteils angeordnet sein. Auch eine Anordnung direkt in der Hohlkammer des Bandteils oder im Kanal eines das Bandteil mit dem Pumpteil verbindenden Schlauchs ist denkbar und möglich.

Vorzugweise weist die Einrichtung eine von der Pumpeinheit (und vom Bandteil) örtlich getrennte Bedieneinheit auf, welche mindestens ein von einem Benutzer betätigbares Bedienelement aufweist, um den Körperkanal zu öffnen und/oder abzusperren. Die Bedieneinheit weist hierbei vorteilhafterweise eine Bedien-Elektronikeinheit auf, welche mit einer Pump-Elektronikeinheit der Pumpeinheit über eine Funkverbindung kommuniziert. Diese Bedieneinheit kann außerhalb des Körpers vorgesehen sein. Auch eine Implantation der Bedieneinheit, und zwar unabhängig vom Ort der Implantation des Bandteils und der Pumpeinheit, ist denkbar und möglich.

Der Luftdrucksensor kann in der (implantierten oder außerhalb des Körpers angeordneten) Bedieneinheit angeordnet sein. Auch eine Anordnung des Luftdrucksensors in der Pumpeinheit ist möglich.

Die Einrichtung kann beispielsweise als künstlicher Urethrasphinkter ausgebildet sein. In anderen Ausführungsformen kann die erfindungsgemäße Einrichtung z.B. als künstlicher Analsphinkter, künstlicher "Sphinkter of oddi" oder künstlicher Verschluss eines Gangs für Gallenflüssigkeit ausgebildet sein.

Das Absperren des Körperkanals kann vollständig oder teilweise (=Verengen) erfolgen.

Weitere Vorteile und Einzelheiten der Erfindung werden im Folgenden anhand der beiliegenden Zeichnung erläutert. In dieser zeigen:
Fig. 1 eine schematische Darstellung einer als künstlicher Urethrasphinkter ausgebildeten Einrichtung, in einem Freigabezustand des Bandteils, in welchem der Harnleiter geöffnet ist;
Fig. 2 eine Darstellung analog Fig. 1 in einem Absperrzustand des Bandteils, in welchem der Harnleiter geschlossen ist;
Fig. 3 und 4 Schrägsichten des freien, also nicht implantierten, Bandteils der Einrichtung in einem geöffneten und geschlossenen Zustand, und zwar entsprechend dem Freigabezustand;
Fig. 5 einen Längsmittelschnitt (parallel zur Längsmittelachse der Durchtrittsöffnung und diese durchsetzend) durch das Bandteil im Zustand von Fig. 4 und
Fig. 6 einen Längsmittelschnitt analog Fig. 5, aber im Absperrzustand des Bandteils.

Ein Ausführungsbeispiel einer erfindungsgemäßen Einrichtung ist in den Figuren dargestellt.

Ein Bandteil 1 der Einrichtung ist ringförmig um das den Körperkanal umgebende Körpergewebe 2, hier die Harnröhre, legbar. Das Bandteil 1 weist eine Hohlkammer 3 auf, die sich in Richtung der Längserstreckung des Bandteils erstreckt, im Ausführungsbeispiel im Wesentlichen über die gesamte Länge des Bandteils. Das Bandteil 1 ist somit schlauchartig ausgebildet mit beidseitig verschlossenen Enden.

An den beiden Enden des Bandteils 1 sind ein erstes und ein zweites Verschlussteil 4, 5 angeordnet. Das erste Verschlussteil 4 besitzt eine Einstecköffnung 4a, in welche eine Zunge 5a des zweiten Verschlussteils 5 einsteckbar und darin verrastbar ist.

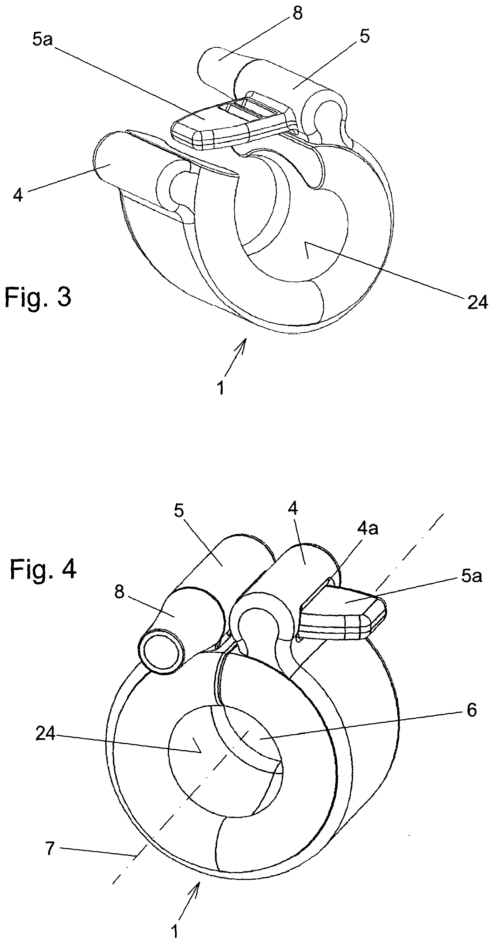

Die Verschlussteile 4, 5 bilden somit einen Verschluss, mit welchem das Bandteil 1 zu einem Ring, insbesondere Kreisring, geschlossen werden kann, vgl. Fig. 3. Im geschlossenen Zustand umschließt das Bandteil 1 eine Durchtrittsöffnung 6 für das den Körperkanal umgebende Körpergewebe 2.

In der Hohlkammer 3 befindet sich ein Fluid, insbesondere eine Flüssigkeit. Die Größe der Durchtrittsöffnung 6 hängt von der Menge des Fluids in der Hohlkammer 3 ab. Durch Einbringen von Fluid in die Hohlkammer 3 kann die Durchtrittsöffnung 6 verkleinert werden. Hierbei wird ein innerer, d.h. der Längsmittelachse 7 der Durchtrittsöffnung 6 benachbarter, flexibler Abschnitt 1a des Bandteils 1 in Richtung zur Längsmittelachse 7 verschoben, wie dies bekannt ist. Durch Abführen von Fluid aus der Hohlkammer 3 kann die Durchtrittsöffnung 6 wieder vergrößert werden.

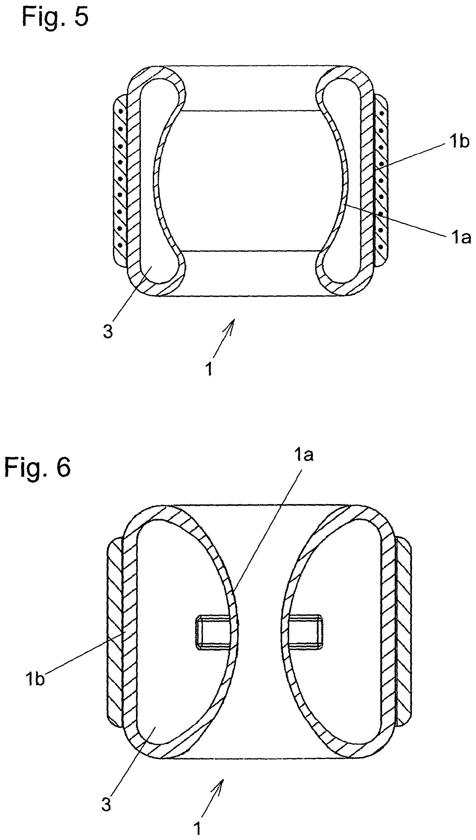

Fig. 5 zeigt den Zustand, in welchem die Durchtrittsöffnung 6 am größten ist (wobei der Druck des Fluids in der Hohlkammer 3 dem Umgebungsdruck entspricht), Fig. 6 zeigt einen mit mehr Fluid befüllten Zustand, insbesondere den maximal mit Fluid befüllten Zustand (wobei der Druck des Fluids in der Hohlkammer 3 über dem Umgebungsdruck liegt). In Fig. 6 sind Faltenbildungen, wie sie sich insbesondere ergeben würden, wenn das Bandteil nicht um den Harnleiter gelegt ist, nicht dargestellt.

Ein von der Längsmittelachse 7 abgelegener Rückenabschnitt 1b des Bandteils 1 kann demgegenüber steifer ausgebildet sein, insbesondere mittels einer Verstärkungslage, wodurch eine Verformung des Rückenabschnitts zumindest weitgehend vermieden werden kann.

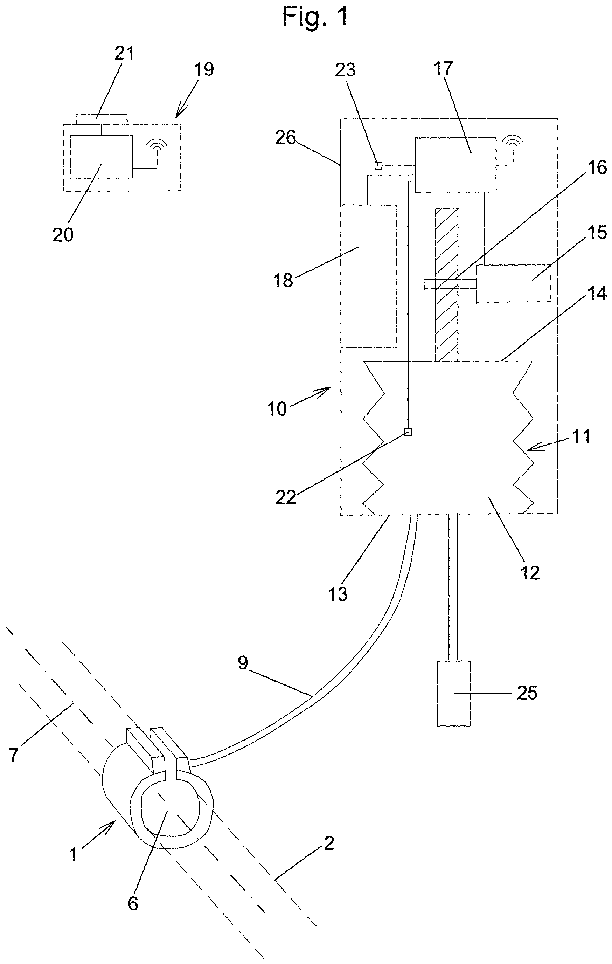

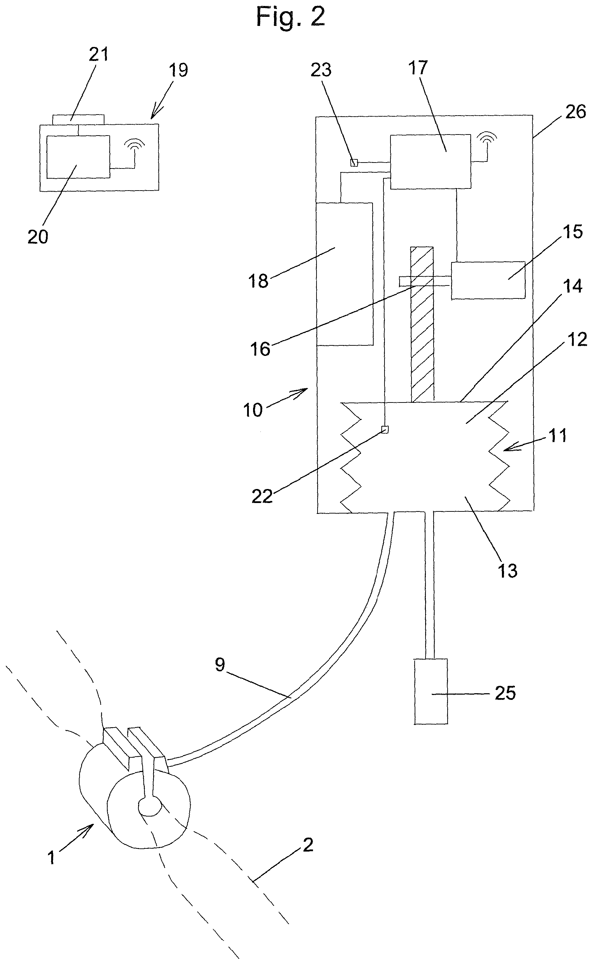

Im geschlossenen Zustand des Bandteils 1 kann dieses also einen Freigabezustand, in welchem der Körperkanal geöffnet ist (vgl. Fig. 1), und einen Absperrzustand, in welchem der Körperkanal geschlossen ist (vgl. Fig. 2), einnehmen. Im Freigabezustand kann der Druck des Fluids in der Hohlkammer 3 beispielsweise dem Umgebungsdruck (=Atmosphärendruck) entsprechen.

In anderen Ausführungsformen der Erfindung könnte der Körperkanal auch nur unterschiedlich stark verengt werden, ohne vollständig geschlossen zu werden. Das Bandteil würde dann also unterschiedliche Verengungszustände einnehmen.

Unterschiedliche Modifikationen der Ausbildung des Bandteils sind denkbar und möglich, so *wäre* es beispielsweise möglich, spezielle am Bandteil 1 angebrachte Verschlussteile überhaupt wegzulassen und die beiden Enden des Bandteils miteinander zu vernähen.

Das Bandteil 1 kann in bekannter Weise aus Silikon bestehen. Auch andere körperverträgliche Materialien sind grundsätzlich einsetzbar.

Das Bandteil 1 steht über einen Schlauch 9 mit einem vom Bandteil 1 örtlich getrennten Pumpteil 11 einer Pumpeinheit 10 in Verbindung, vgl. Fig. 1 und 2. Mittels der Pumpeinheit 10 kann die Menge an Fluid in der Hohlkammer 3 des Bandteils 1 verändert werden.

Im Ausführungsbeispiel ist an einem der Verschlussteile ein Anschlussstutzen 8 angeformt, dessen Innenraum über einen durch das Verschlussteil laufenden Kanal mit der Hohlkammer 3 in Verbindung steht. Ein solcher Anschlussstutzen könnte auch an einer anderen Stelle des Bandteils vorgesehen sein. Der Schlauch 9 ist am Anschlussstutzen 8 angeschlossen.

Das Pumpteil 11 der Pumpeinheit 10 besitzt einen mit Fluid befüllten Aufnahmeraum 12. Im Ausführungsbeispiel wird das Pumpteil 11 von einem Balg gebildet, der von einem Bodenteil 13 und einem Deckelteil, welches ein Stellelement 14 darstellt, verschlossen ist. Ein elektrischer Antrieb 15 wirkt über ein Getriebe 16, beispielsweise Schneckengetriebe, auf das Stellelement 14 ein, um das Volumen des Aufnahmeraums 12 zu ändern. Das Getriebe 16 ist günstigerweise selbsthemmend ausgebildet, sodass eine einmal eingestellte Position des Stellelements 14 ohne Zufuhr von elektrischer Energie an den Antrieb 15 gehalten wird.

Das Pumpteil 11 bildet im Ausführungsbeispiel also gleichzeitig ein Reservoir für das Fluid, mit welchem die Hohlkammer 3 des Bandteils 1 zum Schließen des Körperkanals befüllt wird.

Das Pumpteil 11 könnte beispielsweise auch von einer Kolben-Zylinder-Einheit gebildet werden, wobei das Stellelement 14 vom Kolben dieser Kolben-Zylinder-Einheit gebildet würde.

Die Pumpeinheit 10 besitzt im Weiteren einen elektrischen Antrieb 15 für das Pumpteil 11. Der elektrische Antrieb 15 wird von einer Pump-Elektronikeinheit 17 der Pumpeinheit 10 angesteuert. Die Pump-Elektronikeinheit 17 wird aus einer Batterie 18 der Pumpeinheit 10 mit elektrischer Energie versorgt, ebenso wie der Antrieb 15. Bei der Batterie 18 kann es sich insbesondere um eine wiederaufladbare Batterie (=Akkumulator) handeln. Die Aufladung könnte hierbei berührungslos durch ein außerhalb des Körpers angeordnetes induktiv gekoppeltes Ladegerät erfolgen.

Die Komponenten der Pumpeinheit 10 sind in einem Gehäuse 26 angeordnet. Das Gehäuse 26 besteht aus einem körperverträglichen Material oder ist von einem solchen umhüllt.

Zur Verstellung des Bandteils 1 zwischen dem Absperrzustand und dem Freigabezustand dient eine von der Pumpeinheit 10 örtlich getrennt angeordnete Bedieneinheit 19, die mindestens ein vom Benutzer betätigbares Bedienelement 21, beispielsweise einen Taster, aufweist. Weitere Bedienelemente können vorgesehen sein. Eine Bedien-Elektronikeinheit 20 der Bedieneinheit 19 kommuniziert hierbei mit der Pump-Elektronikeinheit 17 über eine Funkverbindung.

Die Pump-Elektronikeinheit 17 und die Bedien-Elektronikeinheit 20 bilden zusammen die elektrische Steuerung der medizinischen Einrichtung der Erfindung.

Die Bedieneinheit 19 kann außerhalb des Körpers angeordnet sein. Eine Implantation der Bedieneinheit 19 ist denkbar und möglich.

Zur Versorgung der Bedieneinheit 19 mit elektrischer Energie dient eine (in den Figuren nicht dargestellte) Batterie.

Eine separate Bedieneinheit könnte grundsätzlich auch entfallen, wobei mindestens ein vom Benutzer betätigbares Bedienelement an der Pumpeinheit anzuordnen wäre. Dieses müsste entsprechend von außerhalb des Körpers betätigbar sein.

Mit der Pump-Elektronikeinheit 17 ist ein Drucksensor 22 verbunden, der sich im gezeigten Ausführungsbeispiel im mit Fluid gefüllten Aufnahmeraum 12 des Pumpteils 11 befindet. Mittels des Drucksensors 22 ist somit von der elektronischen Steuerung 17 der Druck des Fluids im Aufnahmeraum 12 erfassbar, der dem Druck des Fluids in der Hohlkammer 3 entspricht.

Mit der Pump-Elektronikeinheit 17 ist im Weiteren ein Luftdrucksensor 23 verbunden. Mittels diesem kann von der elektronischen Steuerung 17 der Umgebungsdruck erfasst werden, der dem Atmosphärendruck entspricht. Der Luftdrucksensor 23 könnte auch in der Bedieneinheit 19 angeordnet werden und der von ihm erfasste Wert könnte über die Funkverbindung an die Pump-Elektronikeinheit 17 übertragen werden. Die Auswertung eines mittels des Drucksensors 22 erfassten Druckwerts und eines mittels des Luftdrucksensors 23 erfassten Luftdruckwerts erfolgt im Ausführungsbeispiel also in der Pump-Elektronikeinheit 17. Andererseits könnte von der Pump-Elektronikeinheit 17 auch der mittels des Drucksensors 22 erfasste Druckwert an die in der Bedieneinheit 19 angeordnete Bedien-Elektronikeinheit 20 übertragen werden, um in dieser eine Auswertung durchzuführen. Wenn der Luftdrucksensor 23 nicht in der Bedieneinheit 19 angeordnet ist, könnte in diesem Fall auch der Luftdruckwert von der Pump-Elektronikeinheit 17 an die Bedien-Elektronikeinheit 20 übertragen werden.

Eine Speicherung von erfassten Druckwerten und Luftdruckwerten kann wahlweise in der Pump-Elektronikeinheit 17 oder Bedien-Elektronikeinheit 20 erfolgen.

Die erstmalige Einstellung des Drucks des Fluids in der Hohlkammer, um die Harnröhre zu schließen, kann folgendermaßen durchgeführt werden:
Mittels eines Katheters kann aus einem in definierter Höhe (z.B. 50cm) erhöht gelagerten Gefäß Wasser durch die Harnröhre in die Blase einfließen gelassen werden, wobei der Druck des Fluids in der Hohlkammer 3 erhöht wird, bis kein Wasser mehr durch den Harnleiter fließt. Ein solches Verfahren ist unter der Bezeichnung "Leak-Point-Pressure-Messung" bekannt.

Der so ermittelte Druckwert wird als Referenzwert für den Fluiddruck gespeichert, bei welchem der Körperkanal geschlossen ist. Gleichzeitig wird der dem Atmosphärendruck entsprechende, vom Luftdrucksensor 23 erfasste Luftdruckwert als Referenzwert für den Atmosphärendruck gespeichert, bei welchem der Referenzwert für den Fluiddruck ermittelt worden ist.

Wenn nun der Körperkanal durch Beaufschlagen der Hohlkammer mit diesem Referenzwert für den Fluiddruck geschlossen worden ist und sich bei gleichbleibendem Volumen des Fluids der Atmosphärendruck ändert, so ändert sich auch der vom Drucksensor 22 ausgegebene Druckwert um den gleichen Betrag.

Im Laufe der Zeit kann es beispielsweise durch Osmose oder durch ein Leck zu einer Änderung des Volumens des Fluids kommen. Durch Osmose kann das Fluidvolumen hierbei auch zunehmen. Vor einer jeweiligen Öffnung des Körperkanals wird von der elektronischen Steuerung hierzu überprüft, ob sich der Atmosphärendruck und der Fluiddruck in gleicher Weise geändert haben. Das heißt also, dass mittels der elektronischen Steuerung eine erste Differenz zwischen dem aktuellen Druckwert und dem Referenzwert des Druckwerts und eine zweite Differenz zwischen dem aktuellen Luftdruckwert und dem Referenzwert des Luftdruckwerts ermittelt werden. Die erste Differenz und die zweite Differenz werden mittels der elektronischen Steuerung miteinander verglichen. Sollten die erste Differenz und die zweite Differenz voneinander abweichen, so hat sich das Fluidvolumen geändert.

Wenn die erste Differenz und die zweite Differenz nicht voneinander abweichen, d.h. die erste Differenz und die zweite Differenz stimmen überein, wird der vor dem Öffnen des Körperkanals ermittelte Druckwert als neuer Referenzwert für den Fluiddruck gespeichert und der vor dem Öffnen des Körperkanals erfasste Luftdruckwert wird als neuer Referenzwert für den Atmosphärendruck gespeichert. Beim neuerlichen Schließen des Körperkanals wird der Fluiddruck auf den neuen Referenzwert für den Fluiddruck eingestellt (d.h. auf den Druck, der vor dem Öffnen des Körperkanals vorgelegen ist).

Sollte es aber zu einer Abweichung der ersten Differenz von der zweiten Differenz, und damit zu einer Veränderung des Fluidvolumens, gekommen sein, d.h. die erfassten Werte für den Fluiddruck und den Atmosphärendruck haben sich nicht gleich viel geändert, so wird die Differenz zwischen der Änderung des Atmosphärendrucks und der Änderung des Fluiddrucks ermittelt (also der Unterschied zwischen der ersten Differenz und der zweiten Differenz). Als neuer Referenzwert für den Fluiddruck wird die Summe aus dem vor dem Öffnen des Körperkanals ermittelten Druckwert und der genannten Differenz (=Unterschied zwischen der ersten Differenz und der zweiten Differenz) gespeichert. Der vor dem Öffnen des Körperkanals erfasste Atmosphärendruck wird als neuer Referenzwert für den Atmosphärendruck gespeichert. Beim neuerlichen Schließen des Körperkanals wird der Fluiddruck auf den neuen Referenzwert für den Fluiddruck eingestellt. Die Einstellung des Fluiddrucks wird mittels des Stellelements 14 des Pumpteils 14 vorgenommen, wobei das Volumen des Aufnahmeraums 12 des Pumpteils 11 entsprechend verkleinert oder vergrößert wird. Diese Einstellung des Fluiddrucks bzw. des Fluidvolumens könnte auch als Nachregelung des Fluiddrucks bezeichnet werden.

Dieser Vorgang kann bei jedem Öffnen des Körperkanals wiederholt werden. Es könnten aber auch für einen vorgegebenen Zeitraum die gespeicherten Referenzwerte beibehalten werden und erst nach diesem vorgegebenen Zeitraum beim nächsten Öffnen des Körperkanals die beschriebene Überprüfung und gegebenenfalls Korrektur durchgeführt werden.

Aus jeder Abweichung der ersten Differenz von der zweiten Differenz kann somit auf eine bestimmte Änderung des Fluidvolumens geschlossen werden. Falls sich das Fluidvolumen über ein vorgegebenes Maß geändert hat, also über einen oberen oder unteren Grenzwert hinaus, kann eine Warnung ausgegeben werden.

Ein jeweiliges Öffnen (=Freigeben) des Körperkanals kann vom Benutzer durch einen Druck auf das Bedienelement 21 ausgelöst werden. Ein folgendes Absperren des Körperkanals kann nach einer bestimmten Zeit automatisch erfolgen oder durch den Benutzer ausgelöst werden, beispielsweise durch neuerliche Betätigung des Bedienelements 21 oder durch Betätigung eines anderen Bedienelements. Verschiedene Einstellungen durch den Benutzer können ermöglicht werden, z.B. eine Erhöhung des Schließdrucks des Fluids für eine bestimmte Zeit.

Die erfindungsgemäße Ausbildung ermöglicht auch andere Arten der Steuerung der medizinischen Einrichtung als die zuvor beschriebene. So könnten ein Referenzwert für den Fluiddruck und ein Referenzwert für den Luftdruck gespeichert sein und bei jedem Schließvorgang des Körperkanals könnte der Referenzwert für den Fluiddruck modifiziert um die Differenz zwischen dem aktuellen Messwert des Luftdrucks und dem Referenzwert des Luftdrucks eingestellt werden. Diese Vorgehensweise ist insbesondere dann zweckmäßig, wenn von einem konstanten Volumen des Fluids im System ausgegangen wird. Eine Messung des Fluiddrucks und des Atmosphärendrucks vor dem Öffnen des Körperkanals könnte dann entfallen oder zur Kontrolle durchgeführt werden.

Zum Befüllen des Systems mit Fluid ist in herkömmlicher Weise ein Port 25 vorhanden. Dieser kann beispielsweise über einen Schlauch an das Pumpteil 11 angeschlossen sein.

Anstelle einer Anordnung des Drucksensors 22 im Fluid ist es auch denkbar und möglich, den Drucksensor an einer die Durchtrittsöffnung begrenzenden Oberfläche 24 des Bandteils 1 anzuordnen. Es wird vom Drucksensor damit direkt der auf das Körpergewebe ausgeübte Druck gemessen. Dieser Druck hängt natürlich vom Druck des Fluids in der Hohlkammer 3 ab und ist zumindest weitgehend proportional zu diesem. Abweichungen von der Proportionalität aufgrund von einem nicht linearen Verhalten des Bandteils 1 bei einer Vergrößerung der Hohlkammer 3 liegen vorzugsweise unter 10%.

In Abhängigkeit von der Art des Antriebs 15 könnte es auch möglich sein, aus der Stromaufnahme des Antriebs 15 auf die Größe des Fluiddrucks zu schließen. Ein Drucksensor zur Erfassung des Drucks des Fluids könnte damit entfallen.

**Legende zu den Hinweisziffern:**

| | | | |
|---|---|---|---|
| 1 | Bandteil | 12 | Aufnahmeraum |
| 1a | innerer Abschnitt | 13 | Bodenteil |
| 1b | Rückenabschnitt | 14 | Stellelement |
| 2 | Körpergewebe | 15 | Antrieb |
| 3 | Hohlkammer | 16 | Getriebe |
| 4 | erstes Verschlussteil | 17 | Pump-Elektronikeinheit |
| 4a | Einstecköffnung | 18 | Batterie |
| 5 | zweites Verschlussteil | 19 | Bedieneinheit |
| 5a | Zunge | 20 | Bedien-Elektronikeinheit |
| 6 | Durchtrittsöffnung | 21 | Bedienelement |
| 7 | Längsmittelachse | 22 | Drucksensor |
| 8 | Anschlussstutzen | 23 | Luftdrucksensor |
| 9 | Schlauch | 24 | Oberfläche |
| 10 | Pumpeinheit | 25 | Port |
| 11 | Pumpteil | 26 | Gehäuse |

## Patentansprüche

1. Medizinische Einrichtung zum Verengen oder Absperren eines Körperkanals, umfassend
- ein Bandteil (1), das um das den Körperkanal umgebende Körpergewebe (2) legbar ist und zu einem eine Durchtrittsöffnung (6) für das Körpergewebe umschließenden Ring schließbar ist, wobei das Bandteil (1) eine Hohlkammer (3) aufweist und wobei durch Einbringen eines Fluids in die Hohlkammer (3) die Durchtrittsöffnung (6) verkleinerbar ist, und
- eine Pumpeinheit (10) zur Förderung des Fluids, welche einen von einer elektronischen Steuerung der Einrichtung ansteuerbaren elektrischen Antrieb (15) aufweist, wobei von der elektronischen Steuerung ein dem Druck des Fluids in der Hohlkammer (3) entsprechender oder von diesem abhängender Druckwert erfassbar ist,
**dadurch gekennzeichnet, dass** die Einrichtung einen Luftdrucksensor (23) zur Erfassung des Atmosphärendrucks aufweist, und dass von der elektronischen Steuerung ein Referenzwert des von der elektronischen Steuerung erfassten Druckwerts sowie ein Referenzwert eines mittels des Luftdrucksensors (23) von der elektronischen Steuerung erfassten Luftdruckwerts speicherbar ist, wobei mittels der elektronischen Steuerung der Druck des Fluids in der Hohlkammer (3) zum Verengen oder Absperren des Körperkanals in Abhängigkeit vom gespeicherten Referenzwert des Druckwerts und vom gespeicherten Referenzwert des Luftdruckwerts einstellbar ist.

2. Medizinische Einrichtung nach Anspruch 1, **dadurch gekennzeichnet, dass** die Einrichtung einen Drucksensor (22) aufweist, der im Fluid oder angrenzend an das Fluid oder an einer die Durchtrittsöffnung (6) begrenzenden Oberfläche (24) des Bandteils (1) angeordnet ist.

3. Medizinische Einrichtung nach Anspruch 1 oder 2, **dadurch gekennzeichnet, dass** die Einrichtung eine von der Pumpeinheit (10) räumlich getrennt angeordnete Bedieneinheit (19) aufweist, wobei die Pumpeinheit (10) eine Pump-Elektronikeinheit (17) und die Bedieneinheit (19) eine Bedien-Elektronikeinheit (20) aufweist und die elektronische Steuerung der Einrichtung die Pump-Elektronikeinheit (17) und die Bedien-Elektronikeinheit (20) umfasst und die Pump-Elektronikeinheit (17) und die Bedien-Elektronikeinheit (20) über eine Funkverbindung kommunizieren.

4. Medizinische Einrichtung nach einem der Ansprüche 1 bis 3, **dadurch gekennzeichnet, dass** die Pumpeinheit (10) ein Pumpteil (11) aufweist, das einen mit dem Fluid befüllten Aufnahmeraum (12) aufweist, dessen Volumen mittels des Antriebs (15) veränderbar ist.

5. Medizinische Einrichtung nach Anspruch 4, **dadurch gekennzeichnet, dass** das Pumpteil (11) einen Balg aufweist.

6. Medizinische Einrichtung nach Anspruch 4 oder 5, **dadurch gekennzeichnet, dass** vom Antrieb (15) ein Stellelement (14) des Pumpteils (11) verstellbar ist, wobei sich bei der Verstellung des Stellelements (14) das Volumen des Aufnahmeraums (12) des Pumpteils (11) ändert.

7. Medizinische Einrichtung nach einem der Ansprüche 1 bis 6, **dadurch gekennzeichnet, dass** die Pumpeinheit (10) eine Batterie (18) zur Versorgung des Antriebs (15) mit elektrischer Energie aufweist.

8. Medizinische Einrichtung nach einem der Ansprüche 1 bis 7, **dadurch gekennzeichnet, dass** von der elektronischen Steuerung eine erste Differenz zwischen einem aktuellen Druckwert und dem gespeicherten Referenzwert des Druckwerts und eine zweite Differenz zwischen einem aktuellen Luftdruckwert und dem gespeicherten Referenzwert des Luftdrucks ermittelbar sind, und dass von der elektronischen Steuerung ein Unterschied zwischen der ersten Differenz und der zweiten Differenz ermittelbar ist.

9. Medizinische Einrichtung nach Anspruch 8, **dadurch gekennzeichnet, dass** bei einem Unterschied zwischen der ersten Differenz und der zweiten Differenz als neuer Referenzwert für den Druckwert die Summe aus dem vor einem Öffnen des Körperkanals ermittelten Druckwert und dem Unterschied zwischen der ersten Differenz und der zweiten Differenz speicherbar ist, und dass der vor dem Öffnen des Körperkanals ermittelte Luftdruckwert als neuer Referenzwert für den Luftdruck speicherbar ist.

10. Medizinische Einrichtung nach Anspruch 8, **dadurch gekennzeichnet, dass** bei einer Übereinstimmung der ersten Differenz und der zweiten Differenz als neuer Referenzwert für den Druckwert der vor einem Öffnen des Körperkanals ermittelte Druckwert und als neuer Referenzwert für den Luftdruckwert der vor dem Öffnen des Körperkanals erfasste Luftdruckwert speicherbar sind.

11. Medizinische Einrichtung nach Anspruch 9 oder 10, **dadurch gekennzeichnet, dass** der Druck des Fluids in der Hohlkammer (3) mittels der elektronischen Steuerung auf den neuen Referenzwert der Druckwerts einstellbar ist.

12. Medizinische Einrichtung nach einem der Ansprüche 1 bis 11, **dadurch gekennzeichnet, dass** die medizinische Einrichtung ein künstlicher Urethrasphinkter ist.

13. Verfahren zur Überwachung eines Fluidvolumens einer mit einem Fluid befüllbaren medizinischen Einrichtung zum Absperren und Freigeben eines Körperkanals, umfassend
- ein Bandteil (1), das um das den Körperkanal umgebende Körpergewebe (2) legbar ist und zu einem eine Durchtrittsöffnung (6) für das Körpergewebe umschließenden Ring schließbar ist, wobei das Bandteil (1) eine Hohlkammer (3) aufweist, die zur Verkleinerung der Durchtrittsöffnung (6) mit Fluid befüllbar ist, und
- eine Pumpeinheit (10) zur Förderung des Fluids, welche einen von einer elektronischen Steuerung der Einrichtung ansteuerbaren elektrischen Antrieb (15) aufweist, wobei von der elektronischen Steuerung ein dem Druck des Fluids in der Hohlkammer (3) entsprechender oder von diesem abhängender Druckwert erfassbar ist, **dadurch gekennzeichnet, dass**
die Einrichtung einen Luftdrucksensor (23) zur Erfassung des Atmosphärendrucks aufweist, und von der elektronischen Steuerung ein vom Luftdrucksensor (23) ausgegebener Luftdruckwert erfassbar ist, wobei in der elektronischen Steuerung ein Referenzwert des von der elektronischen Steuerung erfassten Druckwerts sowie ein Referenzwert des mittels des Luftdrucksensors (23) von der elektronischen Steuerung erfassten Luftdruckwerts gespeichert werden, und wobei von der elektronischen Steuerung vor dem jeweiligen Freigeben des Körperkanals ein aktueller Druckwert und mittels des Luftdrucksensors (23) ein aktueller Luftdruckwert erfasst werden, und wobei mittels der elektronischen Steuerung eine erste Differenz zwischen dem aktuellen Druckwert und dem Referenzwert des Druckwerts und eine zweite Differenz zwischen dem aktuellen Luftdruckwert und dem Referenzwert des Luftdruckwerts gebildet werden und durch Vergleichen der ersten Differenz und der zweite Differenz mittels der elektronischen Steuerung auf eine Veränderung des Fluidvolumens geschlossen wird.

14. Verfahren nach Anspruch 13, **dadurch gekennzeichnet, dass** aus einer Abweichung der ersten Differenz von der zweiten Differenz auf eine Änderung des Fluidvolumens geschlossen wird.

15. Verfahren nach Anspruch 14, **dadurch gekennzeichnet, dass**, bei einer Änderung des Fluidvolumens über einen oberen und/oder unteren Grenzwert hinaus, eine Warnung ausgegeben wird.

## Claims

1. A medical device for the narrowing or blocking of an anatomical channel, comprising
- a band part (1) which can be placed around the body tissue (2) surrounding the anatomical channel and is closeable to form a ring enclosing a through opening (6) for the body tissue, wherein the band part (1) has a hollow chamber (3) and wherein the through opening (6) can be reduced in size by the introduction of a fluid into the hollow chamber (3), and
- a pumping unit (10), for conveying the fluid, which has an electric drive (15) controllable by an electronic control means of the device, wherein a pressure value corresponding to the pressure of the fluid in the hollow chamber (3) or dependent thereon can be detected by the electronic control means,
**characterised in that** the device has an air pressure sensor (23) for detecting the atmospheric pressure, and **in that** a reference value of the pressure value detected by the electronic control means as well as a reference value of an air pressure value detected by the electronic control means by means of the air pressure sensor (23) can be stored by the electronic control means, wherein dependent on the stored reference value of the pressure value and on the stored reference value of the air pressure value, the pressure of the fluid in the hollow chamber (3) is adjustable by means of the electronic control means to narrow or block the anatomical channel.

2. A medical device according to claim 1, **characterised in that** the device has a pressure sensor (22) which is arranged in the fluid or adjacent to the fluid or at a surface (24), bounding the through opening (6), of the band part (1).

3. A medical device according to claim 1 or 2, **characterised in that** the device has an operating unit (19) arranged spatially separated from the pumping unit (10), wherein the pumping unit (10) has a pump electronic unit (17) and the operating unit (19) has an operating electronic unit (20) and the electronic control means of the device comprises the pump electronic unit (17) and the operating electronic unit (20) and the pump electronic unit (17) and the operating electronic unit (20) communicate via a radio connection.

4. A medical device according to any one of claims 1 to 3, **characterised in that** the pumping unit (10) has a pump part (11) which has a receiving chamber (12) which is filled with the fluid and whose volume is alterable by means of the drive (15).

5. A medical device according to claim 4, **characterised in that** the pump part (11) has bellows.

6. A medical device according to claim 4 or 5, **characterised in that** an adjustment element (14) of the pump part (11) is adjustable by the drive (15), wherein the volume of the receiving chamber (12) of the pump part (11) alters upon adjustment of the adjustment element (14).

7. A medical device according to any one of claims 1 to 6, **characterised in that** the pumping unit (10) has a battery (18) to supply the drive (15) with electrical energy.

8. A medical device according to any one of claims 1 to 7, **characterised in that** a first quantitative difference between an actual pressure value and the stored reference value of the pressure value and a second quantitative difference between an actual air pressure value and the stored reference value of the air pressure can be determined by the electronic control means, and **in that** a difference between the first quantitative difference and the second quantitative difference can be determined by the electronic control means.

9. A medical device according to claim 8, **characterised in that** in the event of a difference between the first quantitative difference and the second quantitative difference, the sum of the pressure value determined before an opening of the anatomical channel and of the difference between the first quantitative difference and the second quantitative difference can be stored as a new reference value for the pressure value, and **in that** the air pressure value determined before the opening of the anatomical channel can be stored as a new reference value for the air pressure.

10. A medical device according to claim 8, **characterised in that** in the event of an agreement of the first quantitative difference and the second quantitative difference, the pressure value determined before an opening of the anatomical channel can be stored as a new reference value for the pressure value and the air pressure value detected before the opening of the anatomical channel can be stored as a new reference value for the air pressure value.

11. A medical device according to claim 9 or 10, **characterised in that** the pressure of the fluid in the hollow chamber (3) is adjustable to the new reference value of the pressure value by means of the electronic control means.

12. A medical device according to any one of claims 1 to 11, **characterised in that** the medical device is an artificial urethral sphincter.

13. A method of monitoring a fluid volume of a medical device, fillable with a fluid, for blocking and unblocking an anatomical channel, comprising
- a band part (1) which can be placed around the body tissue (2) surrounding the anatomical channel and is closeable to form a ring enclosing a through opening (6) for the body tissue, wherein the band part (1) has a hollow chamber (3) fillable with fluid to reduce the size of the through opening (6), and
- a pumping unit (10), for conveying the fluid, which has an electric drive (15) controllable by an electronic control means of the device, wherein a pressure value corresponding to the pressure of the fluid in the hollow chamber (3) or dependent thereon can be detected by the electronic control means,
**characterised in that** the device has an air pressure sensor (23) for detecting the atmospheric pressure, and an air pressure value emitted by the air pressure sensor (23) can be detected by the electronic control means, wherein a reference value of the pressure value detected by the electronic control means as well as a reference value of the air pressure value detected by the electronic control means by means of the air pressure sensor (23) are stored in the electronic control means, and wherein an actual pressure value is detected by the electronic control means before the specific unblocking of the anatomical channel and an actual air pressure value is detected by the electronic control means by means of the air pressure sensor (23), and wherein a first quantitative difference between the actual pressure value and the reference value of the pressure value and a second quantitative difference between the actual air pressure value and the reference value of the air pressure value are formed by means of the electronic control means and through comparison of the first quantitative difference and the second quantitative difference by means of the electronic control means there is inferred an alteration in the fluid volume.

14. A method according to claim 13, **characterised in that** a change in the fluid volume is inferred from a divergence of the first quantitative difference from the second quantitative difference.

15. A method according to claim 14, **characterised in that** an alarm is emitted in the event of a change in the fluid volume beyond an upper and/or lower threshold value.

## Revendications

1. Dispositif médical pour rétrécir ou fermer un canal corporel, comprenant
- une partie de bande (1) qui peut être placée autour du tissu corporel (2) entourant le canal corporel et qui peut être fermée en un anneau entourant une ouverture de passage (6) pour le tissu corporel, la partie de bande (1) présentant une chambre creuse (3) et l'ouverture de passage (6) pouvant être réduite par introduction d'un fluide dans la chambre creuse (3), et
- une unité de pompage (10) pour le déplacement du fluide, laquelle présente un entraînement électrique (15) pouvant être commandé par une commande électronique du dispositif, une valeur de pression correspondant à la pression du fluide dans la chambre creuse (3), ou dépendant de celle-ci, pouvant être détectée par la commande électronique,
**caractérisé en ce que** le dispositif présente un capteur de pression air (23) pour détecter la pression atmosphérique, et **en ce qu'**une valeur de référence de la valeur de pression détectée par la commande électronique ainsi qu'une valeur de référence d'une valeur de pression d'air détectée par la commande électronique au moyen du capteur de pression d'air (23) peuvent être mémorisées par la commande électronique, la pression du fluide dans la chambre creuse (3) pouvant être réglée au moyen de la commande électronique pour rétrécir ou fermer le canal corporel en fonction de la valeur de référence mémorisée de la valeur de pression et de la valeur de référence mémorisée de la valeur de pression d'air.

2. Dispositif médical selon la revendication 1, **caractérisé en ce que** le dispositif présente un capteur de pression (22) qui est disposé dans le fluide ou de manière adjacente au fluide ou sur une surface (24) de la partie de bande (1) délimitant l'ouverture de passage (6).

3. Dispositif médical selon la revendication 10u 2, **caractérisé en ce que** le dispositif présente une unité de commande (19) disposée de manière séparée dans l'espace de l'unité de pompage (10), l'unité de pompage (10) présentant une unité électronique de pompage (17), l'unité de commande (19) présentant une unité électronique de commande (20), et la commande électronique du dispositif comprenant l'unité électronique de pompage (17) et l'unité électronique de commande (20), et l'unité électronique de pompage (17) et l'unité électronique de commande (20) communiquant par une liaison radio.

4. Dispositif médical selon l'une des revendications 1 à 3, **caractérisé en ce que** l'unité de pompage (10) présente une partie de pompage (11) qui présente un espace de réception (12) rempli du fluide et dont le volume peut être modifié au moyen de l'entraînement (15).

5. Dispositif médical selon la revendication 4, **caractérisé en ce que** la partie de pompage (11) présente un soufflet.

6. Dispositif médical selon la revendication 4 ou 5, **caractérisé en ce qu'**un élément de réglage (14) de la partie de pompage (11) peut être réglé par l'entraînement (15), le volume de l'espace de réception (12) de la partie de pompage (11) se modifiant lors du réglage de l'élément de réglage (14).

7. Dispositif médical selon l'une des revendications 1 à 6, **caractérisé en ce que** l'unité de pompage (10) présente une batterie (18) pour alimenter l'entraînement (15) en énergie électrique.

8. Dispositif médical selon l'une des revendications 1 à 7, **caractérisé en ce qu'**une première différence entre une valeur de pression actuelle et la valeur de référence mémorisée de la valeur de pression, et une deuxième différence entre une valeur de pression d'air actuelle et la valeur de référence mémorisée de la pression d'air peuvent être déterminées par la commande électronique, et **en ce qu'**une différence entre la première différence et la deuxième différence peut être déterminée par la commande électronique.

9. Dispositif médical selon la revendication 8, **caractérisé en ce que,** en cas de différence entre la première différence et la deuxième différence, la somme de la valeur de pression déterminée avant l'ouverture du canal corporel et de la différence entre la première différence et la deuxième différence peut être mémorisée en tant que nouvelle valeur de référence pour la valeur de pression, et **en ce que** la valeur de pression de l'air déterminée avant l'ouverture du canal corporel peut être mémorisée en tant que nouvelle valeur de référence pour la pression de l'air.

10. Dispositif médical selon la revendication 8, **caractérisé en ce que,** en cas de concordance entre la première différence et la deuxième différence, la valeur de pression déterminée avant une ouverture du canal corporel peut être mémorisée en tant que nouvelle valeur de référence pour la valeur de pression, et la valeur de pression de l'air déterminée avant l'ouverture du canal corporel peut être mémorisée en tant que nouvelle valeur de référence pour la valeur de pression de l'air.

11. Dispositif médical selon la revendication 9 ou 10, **caractérisé en ce que** la pression du fluide dans la chambre creuse (3) peut être réglée au moyen de la commande électronique sur la nouvelle valeur de référence de la valeur de pression.

12. Dispositif médical selon l'une des revendications 1 à 11, **caractérisé en ce que** le dispositif médical est un sphincter urétral artificiel.

13. Procédé de surveillance d'un volume de fluide d'un dispositif médical pouvant être rempli d'un fluide pour fermer et libérer un canal corporel, comprenant
- une partie de bande (1) qui peut être placée autour du tissu corporel (2) entourant le canal corporel et qui peut être fermée en un anneau entourant une ouverture de passage (6) pour le tissu corporel, la partie de bande (1) présentant une chambre creuse (3) qui peut être remplie de fluide pour réduire l'ouverture de passage (6), et
- une unité de pompage (10) pour le déplacement du fluide, laquelle présente un entraînement électrique (15) pouvant être commandé par une commande électronique du dispositif, une valeur de pression correspondant à la pression du fluide dans la chambre creuse (3), ou dépendant de celle-ci, pouvant être détectée par la commande électronique, **caractérisé en ce que**
le dispositif présente un capteur de pression d'air (23) pour détecter la pression atmosphérique, et une valeur de pression d'air donnée par le capteur de pression d'air (23) peut être détectée par la commande électronique, une valeur de référence de la valeur de pression détectée par la commande électronique ainsi qu'une valeur de référence de la valeur de pression d'air détectée par la commande électronique au moyen du capteur de pression d'air (23) étant mémorisées dans la commande électronique, et, avant chaque libération du canal corporel, une valeur de pression actuelle étant détectée par la commande électronique ainsi qu'une valeur de pression d'air actuelle au moyen du capteur de pression d'air (23), et une première différence entre la valeur de pression actuelle et la valeur de référence de la valeur de pression et une deuxième différence entre la valeur de pression d'air actuelle et la valeur de référence de la valeur de pression d'air étant formées au moyen de la commande électronique, et une modification du volume de fluide étant déduite par comparaison de la première différence et de la deuxième différence au moyen de la commande électronique.

14. Procédé selon la revendication 13, **caractérisé en ce qu'**une modification du volume de fluide est déduite d'un écart entre la première différence et la deuxième différence.

15. Procédé selon la revendication 14, **caractérisé en ce qu'un** avertissement est émis en cas de modification du volume de fluide au-delà d'une valeur limite supérieure et/ou inférieure.
